# EUROPEAN PATENT SPECIFICATION

(11) **EP 4 210 733 B1**
(45) Date of publication and mention of the grant of the patent: **06.11.2024**
(21) Application number: 21770243.0
(22) Date of filing: 02.09.2021
(51) Int. Cl.: A61K 39/00, C07K 16/30, C07K 14/725, C07K 16/44, C12N 15/63, A61P 35/00

(54) **SYSTEM FOR INDUCIBLE EXPRESSION OF AN ADAPTER IN IMMUNE CELLS**
SYSTEM ZUR INDUZIERBAREN EXPRESSION EINES ADAPTERS IN IMMUNZELLEN
SYSTÈME D'EXPRESSION INDUCTIBLE D'UN ADAPTATEUR DANS DES CELLULES IMMUNITAIRES

(30) Priority: 04.09.2020 EP 20194509
(43) Date of publication of application: 19.07.2023
(73) Proprietor: Miltenyi Biotec B.V. & Co. KG, 51429 Bergisch Gladbach (DE)
(72) Inventor: KOTTER, Bettina, 51429 Bergisch Gladbach (DE); MITTELSTAET, Joerg, 51429 Bergisch Gladbach (DE); WEBSTER, Brian, 51429 Bergisch Gladbach (DE); HEEMSKERK, Bianca, 51429 Bergisch Gladbach (DE); KAISER, Andrew, 51429 Bergisch Gladbach (DE)
(74) Representative: Biervert, Christian
(86) International application number: PCT/EP2021/074307
(87) International publication number: WO 2022/049217

(56) References cited:
- WO-A2-2012/082841
- R. SAKEMURA ET AL: "A Tet-On Inducible System for Controlling CD19-Chimeric Antigen Receptor Expression upon Drug Administration", CANCER IMMUNOLOGY RESEARCH, vol. 4, no. 8, 21 June 2016 (2016-06-21), US, pages 658 - 668, XP055513121, ISSN: 2326-6066, DOI: 10.1158/2326-6066.CIR-16-0043
- XINGJIAN GU ET AL: "Development of Inducible CD19-CAR T Cells with a Tet-On System for Controlled Activity and Enhanced Clinical Safety", INTERNATIONAL JOURNAL OF MOLECULAR SCIENCES, vol. 19, no. 11, 3 November 2018 (2018-11-03), pages 3455, XP055555288, DOI: 10.3390/ijms19113455
- ARNDT CLAUDIA ET AL: "Adaptor CAR Platforms-Next Generation of T Cell-Based Cancer Immunotherapy", vol. 12, no. 5, 21 May 2020 (2020-05-21), pages 1302, XP055783293, Retrieved from the Internet <URL:https://www.ncbi.nlm.nih.gov/pmc/articles/PMC7281723/pdf/cancers-12-01302.pdf> DOI: 10.3390/cancers12051302
- AMBROSE CHRISTINE ET AL: "CD19-targeting CAR T cells potently redirected to kill solid tumor cells", BIORXIV, 26 March 2020 (2020-03-26), XP055783671, Retrieved from the Internet <URL:https://www.biorxiv.org/content/10.1101/2020.03.25.007658v1.full.pdf> [retrieved on 20210309], DOI: 10.1101/2020.03.25.007658
- TRISTÁN-MANZANO MARÍA ET AL: "Externally-Controlled Systems for Immunotherapy: From Bench to Bedside", vol. 11, 1 January 2020 (2020-01-01), XP055783095, Retrieved from the Internet <URL:https://www.ncbi.nlm.nih.gov/pmc/articles/PMC7498544/pdf/fimmu-11-02044.pdf> DOI: 10.3389/fimmu.2020.02044

## Description

### Field of the invention

The present invention generally relates to the field of chimeric antigen receptors (CARs) expressed on immune cells, in particular to a combination of the adapter CAR (anti-tag CAR) technology and the regulated expression of the corresponding tagged adapter molecule.

### Background of the invention

Adoptive transfer of CAR immune cells such as CAR T cells has demonstrated remarkable success in treatment of hematological malignancies. However, lack of control of CAR immune cell function and consequent excessive inflammation in patients can result in severe side effects especially when targeting tumor-associated rather than tumor-specific antigens. Thus, temporal, tunable and spatial control of CAR activity is of major importance.

Adapter CAR immune cells such as adapter CAR T cells are based on the dissociation of the target antigen recognition and activation domain into two complementary parts, thereby presenting a further strategy to enhance safety and flexibility of CAR immune cell systems. Anti-tag CAR immune cells do not directly recognize the tumor antigen but immune cell specificity is granted to a tag fused to an antigen-binding molecule, referred to as an adapter or adapter molecule. Such "Universal" CAR systems (or adapter CAR (adCAR) systems) that indirectly bind to target cells via adapters are described e.g. in WO2012082841A2, WO2013044225A1 and WO2016030414A1. The recognition of the antigen is strictly dependent on the presence of the adapter molecule giving control and the possibility for temporal on-/off-switching of CAR-mediated functions by attenuating adapter molecule administration. Furthermore, the magnitude of response can be fine-tuned by adjusting adapter concentrations. However, as functionality is dependent on the presence of the adapter molecule, patients are subjected to routine injection of the adapter molecule, which might be associated with side effects and elevated stress for the patient. Moreover, the centralized GMP-compliant production, formulation, storage, and transport of adapter molecules is time- and cost-intensive. The most relevant challenge limiting the potency of adapter CAR immune cells, is the restricted tissue penetration and heterogenous distribution of adapter molecules in tumors upon systemic administration.

Ambrose et al developed a CAR-CD19 T cell that constitutively secretes a CD19-anti-Her2 bridging protein. This cell therapy strategy exploits the ability of CD 19-targeting CAR T cells to interact with CD19 on normal B cells to drive expansion, persistence and fitness. The secreted bridging protein potently binds to Her2-positive tumor cells, mediating CAR-CD19 T cell cytotoxicity *in vitro* and *in vivo* (Doi: 10.1101/2020.03.25.007658).

WO2017075537A1 discloses a cell comprising a constitutive expression construct encoding a fusion protein comprising (a) an antigen-binding protein or fragment that binds a tumor antigen; and (b) a polypeptide target for a cellular therapeutic, antibody, or antibody-drug conjugate.

WO2018156802A1 discloses a cell comprising a constitutive expression construct encoding a fusion protein comprising (a) an antigen-binding protein or fragment that binds a tumor antigen; and (b) an anti-idiotype antibody or fragment, or an anti-idiotype peptide, that binds an antigen binding domain of a cellular therapeutic, antibody, or antibody-drug conjugate.

In WO2019199689A1 engineered immune cells are disclosed that comprise a single viral vector comprising both a first polynucleotide comprising a constitutive promoter operably linked to a nucleic acid encoding at least one transgene, e.g. a CAR; and a second polynucleotide comprising an inducible promoter operably linked to a nucleic acid encoding an effector.

There is a need in the art for an improved or alternative adapter CAR immune cell system comprising adapterCARs and adapters expressed by immune cells.

### Brief description of the invention

To overcome the restrictions associated with the centralized GMP- compliant production and local delivery and injection of adapter molecules into patients, immune cells such as CAR T cells or tumor-infiltrating T cells (TILs), which traffic to the tumor, could be engineered to express the adapter suited for an adapter CAR in a inducer-dependent way thereby functioning as vehicle for in situ delivery of the adapter. The inducible gene expression system may be an "antigen-activated inducible gene expression system", i.e. the inducible expression system may be activated in a cell having said inducible gene expression system, when an antigen/ligand is bound by directly or upon MHC presentation by a receptor of the cell such as a CAR or an TCR. Said binding of the antigen/ligand to the receptor may induce a signal cascade within the cell that subsequently may lead to the induction of the expression of the introduced gene (or transgene), herein the adapter. Alternatively, the inducible gene expression system may be a drug-inducible gene expression system, i.e. the inducible gene expression system may be activated in a cell having said inducible gene expression system, when a drug, e.g. a synthetic drug such as tamoxifen may be introduced to the cell. Said drug in the cell may bind to a synthetic transcription factor and subsequently may lead to the induction of the expression of the transgene, herein the adapter. Both kinds of inducible gene expression systems may be used in a one cell and/or two cell system. While constitutive expression of adapters by the cell vehicle does not allow to tune adapter expression and thereby control adapter CAR

cell function in the patient, drug dependent expression control of the adapter molecule by the "vehicle cell" allows temporal and tunable control of adapter molecule secretion, enabling e.g.:
I) controlled on- and off-switching of CAR immune cell activity by restricting CAR cell immune activity to the temporally controlled secretion of the adapter
ii) precise adjustment of the adapter concentration at the tumor site and thus fine-tuning of CAR immune cell activity.

Further the "antigen-activated inducible gene expression system", in case of locally confined antigens, offers the advantage that adapter secretion is limited to areas in which the antigen is expressed (e.g. solid tumors) as activation-induced signaling is dependent on T cell engagement by the cognate antigen (on signaling of the TCR/CD3 pathway), and thereby offers improved spatial regulation of adapter concentration. In contrast, constitutive expression of adapters may result in systemic release thereby increasing the risk of undesired systemic toxicities.

### Brief description of the drawings

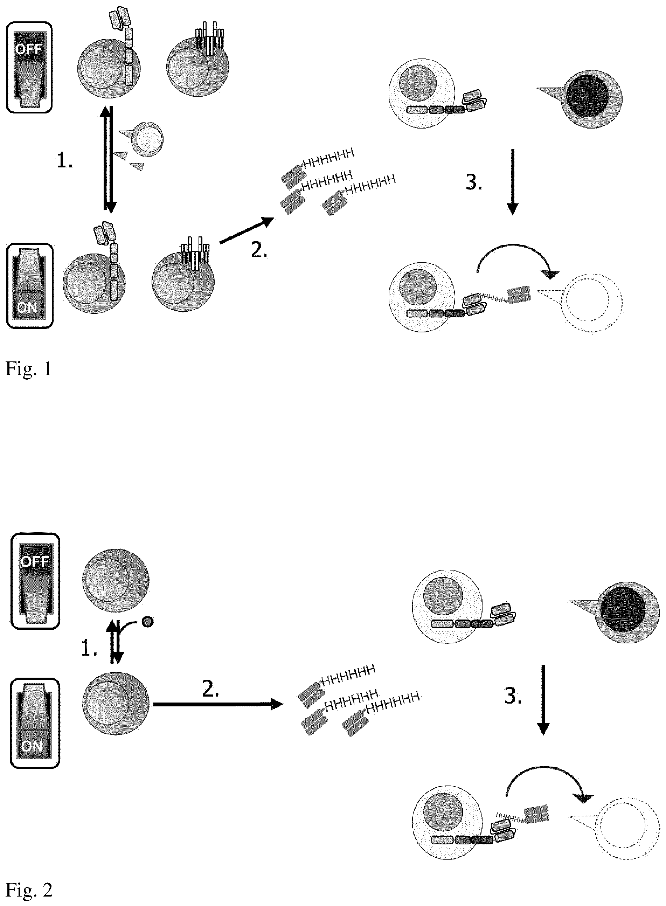

Fig 1: Schematic drawing of the activation-induced system for adapter secretion
   Immune cells are modified with an activation-inducible cassette, i.e. gene expression system (referred to as activation-inducible cells), whereby a promoter responsive to TCR/CAR-mediated signaling drives expression of an adapter molecule. These cells can be selected on the basis of their reactivity to tumor antigens, oncoviral antigens, or against tumor neo-antigens. Alternatively, T cells can be transduced with a CAR or TCR specific for any of the above mentioned antigen categories. Upon TCR/CAR recognition of the TAA, oncoviral, or tumor neo-antigen, the TCR-reactive promoter drives expression of an anti-tumor adapter molecule. These tagged adapter molecules are specific for a tumor antigen. The tag moiety (in this case for example: 6xHis Tag) of the adapter molecule is recognized by the extracellular recognition domain of the adapter CAR (in this case: anti-His₆ specific CAR), which is expressed by a second immune cell. The tagged adapter molecule constitutes a bridging molecule redirecting the adapter CAR cells to the tumor. Consequently, tumor cells are lysed by the adapter CAR cell.
Fig 2: Schematic drawing of the drug-induced system for adapter secretion
   Immune cells are modified with a drug-inducible gene expression cassette (referred to as drug-inducible cells). In the "off" state those inducible cells cannot be distinguished from unmodified immune cells. Upon the administration of the drug as an inducer, the inducible expression cassette is activated and tagged adapter molecules are expressed and secreted by the immune cell. The concentration of secreted tagged adapter molecule can be adjusted by the inducer drug concentration. These tagged adapter molecules are specific for a tumor antigen. The tag moiety (in this case: 6xHis Tag) of the adapter molecule is recognized by the extracellular recognition domain of the adapter CAR (in this case: anti-His₆ specific CAR), which is expressed by a second immune cell. The tagged adapter molecule constitutes a bridging molecule redirecting the adapter CAR cells to the tumor. Consequently, tumor cells are lysed by the adapter CAR cell. Anti-tumor activity of His-Adapter CAR T cells is dependent on the presence of a His-tagged, tumor-specific adapter molecule. Inducible anti-CD19 Fab T cells produce the adapter molecule upon induction at the tumor site.

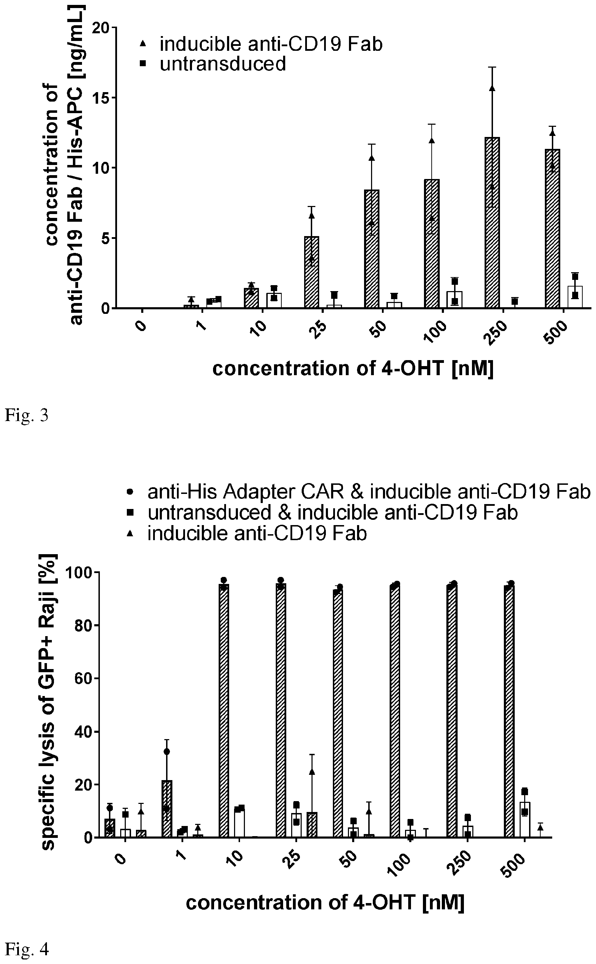

Fig 3: Induction of anti-CD19 Fab-His₆ secretion by primary T cells.
   T cells were transduced with the inducible anti-CD19-Fab construct and cultured in the presence of distinct concentrations of 4-OHT for 48 h. CD19⁺ Raji cells were subsequently stained using T cell supernatant and anti-His-APC as secondary antibody. The determined mean fluorescent intensity was correlated to the Fab concentration by extrapolation from a standard curve. Induction of His₆-tagged anti-CD19-Fab secretion by inducible T cells is strictly dependent on the presence of the inducer drug 4-OHT. With increasing concentrations of 4-OHT, increasing amounts of His₆-tagged anti-CD19-Fab is detected in the supernatant of the T cell culture.
Fig 4: Cytolytic activity of anti-His Adapter CAR T cells in co-culture with Raji cells is dependent on the presence of inducible anti-CD19 Fab T cells and the inducer drug 4-OHT. Anti-His Adapter CAR T cells were co-cultured with CD19⁺ Raji cells at an E:T ratio of 2:1 in the presence of 1×10⁴ anti-CD19 Fab inducible T cells that secrete the adapter molecule, a His-tagged CD19 Fab, upon induction with the inducer drug 4-OHT (• symbol). Specific lysis of CD19⁺ Raji cells was only detected in co-cultures of CD19⁺ Raji cells, adapter CAR T cells and inducible T cells in the presence of at least 1 nM 4-OHT. Maximal specific lysis was obtained following addition of at least 10 nM 4.OHT. A co-culture of untransduced T cells and CD19⁺ Raji in the presence of anti-CD19 Fab inducible T cells (■ symbol) as well as a co-culture of inducible anti-CD19 Fab T cells and CD19⁺ Raji (▲ symbol) served as negative control both indicating that lysis of tumor cells cannot be induced by the adapter molecule itself but requires the simultaneous presence of anti-His adapter CAR T cells. The specific lysis of tumor cells was determined by the quantification of living Raji cells via MACSQuant^{®} Analyzer 6 days post assay initiation.

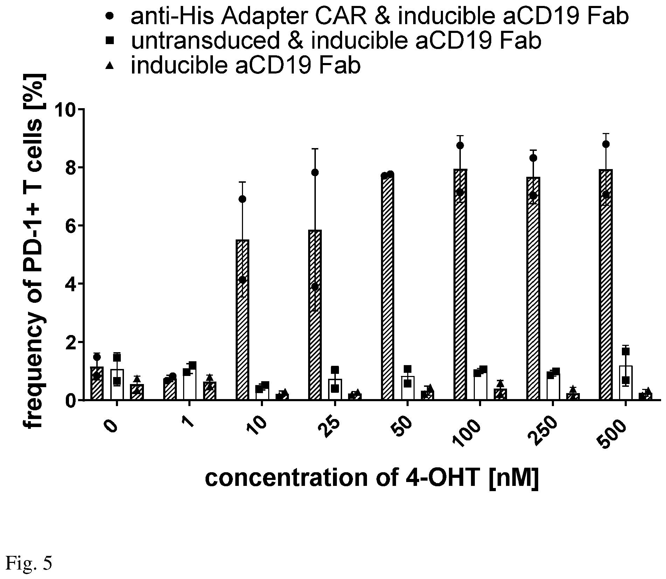

Fig 5: The activation of adapter CAR T cells in co-culture with Raji cells is dependent on the presence of inducible anti-CD19 Fab T cells and the inducer drug 4-OHT. Anti-His Adapter CAR T cells were co-cultured with CD19⁺ Raji cells at an E:T ratio of 2:1 in the presence of 1×10⁴ inducible T cells that secrete the adapter molecule, a His-tagged CD19 Fab, upon induction with the inducer drug 4-OHT (• symbol). PD-1 expression on T cells was only detected in co-cultures of CD19⁺ Raji cells, adapter CAR T cells and inducible T cells in the presence of at least 10 nM 4-OHT and the frequency of PD-1 positive T cells increased with the concentration of 4-OHT. Maximal frequency of PD-1 positive cells was obtained following addition of at least 50 nM 4.OHT. A co-culture of untransduced T cells and CD19⁺ Raji in the presence of anti-CD19 Fab inducible T cells (■ symbol) as well as a co-culture of inducible anti-CD19 Fab T cells and CD19⁺ Raji (▲ symbol) served as negative control both indicating that activation (as shown by PD-1 expression) of T cells cannot be induced by the adapter molecule itself but requires the simultaneous presence of anti-His adapter CAR T cells. The frequency of PD-1 expressing T cells were determined by staining the co-cultures with anti-PD-1-PE-Vio770 on day 2 post assay initiation.

### Detailed description of the invention

In a first aspect the invention provides a system (or a combination of nucleic acids) for inducible expression of an adapter (or adapter molecule or tagged polypeptide) in immune cells comprising
a) an inducible gene expression system comprisingI) a first nucleic acid comprising an inducible promoter operably linked to a second nucleic acid
   II) said second nucleic acid encoding an adapter comprising
   i) a first (poly)peptide, wherein said first (poly)peptide comprises an antigen binding domain that binds specifically to an antigen,
   ii) a second (poly)peptide, wherein said second (poly)peptide binds to an antigen binding domain of a chimeric antigen receptor (CAR),
b) a third nucleic acid encoding said CAR specific for said second polypeptide of said adapter, wherein said CAR comprises
   i) said antigen binding domain specific for said second (poly)peptide of said adapter
   ii) a transmembrane domain
   iii) an intracellular signaling domain.

Said CAR specific for said second polypeptide of said adapter may be expressed constitutively in a cell or said expression may be inducible in a cell.

Said antigen may be an antigen expressed on the surface of a target cell.

Said antigen may be a tumor associated antigen (TAA) and said target cell may be a tumor cell. Said antigen may be expressed by a cell of the tumor microenvironment.

Said antigen may be an infectious pathogen-associated antigen (e.g., from human immunodeficiency virus or other viruses) and said target cell may be a pathogen-infected cell.

Said second (poly)peptide of the adapter that may bind to an antigen binding domain of a chimeric antigen receptor (CAR) may also be referred to as a "tag" and said CAR specific for said second polypeptide of said adapter may also be referred to as anti-tag CAR or adapterCAR (adCAR) or universal CAR.

The tag may be a (poly)peptide comprising at least 4 amino acids

The tag may be a peptide comprising at least 4 amino acids and not more than 8, 10, 15, 20, 25 or 30 amino acids.

The tag may comprise an epitope of a protein.

The tag may comprise an epitope of a protein naturally occurring in a subject or in a circulatory system of a subject.

The tag may comprise an epitope of a neo-antigen, wherein said epitope comprises the mutation of the antigen.

The tag may comprise an epitope of a neo-antigen as may occur in tumorigenesis, wherein said epitope comprises the mutation of the antigen.

The tag may comprise an epitope of a neo-antigen, wherein said epitope comprises the mutation of the antigen, wherein said antigen may be a protein naturally occurring in a subject or in a circulatory system of a subject.

The tag may be a (poly)peptide that does not naturally occur in a subject.

The tag may be a (poly)peptide that does not naturally occur in the circulatory system of a subject.

The tag may be a (poly)peptide that does not naturally occur in the circulatory system of a healthy subject.

The tag may be e.g. one of the following peptides: c-Myc-tag, Strep-Tag II, Flag-Tag, Polyhistidine-tag, Avi-Tag, Calmodulin binding protein-Tag, Yol-tag (derived from alpha-tubulin), E-tag, HA-Tag, S-tag, SBP-tag or V5 tag.

The c-Myc-tag may comprise SEQ ID NO:1.

The Strep-tag II may comprise SEQ ID NO:2 or SEQ ID NO:3

The Flag-tag may comprise SEQ ID NO:4.

The Polyhistidine-tag may comprise SEQ ID NO:5.

The Polyhistidine-tag may comprise SEQ ID NO:5.

The Avi-tag may comprise SEQ ID NO:6.

The Calmodulin binding protein -tag may comprise SEQ ID NO:7.

The E-tag may comprise SEQ ID NO:8.

The HA-tag may comprise SEQ ID NO:9.

The S-tag may comprise SEQ ID NO: 10.

The SBP-tag may comprise SEQ ID NO: 11.

The V5-tag may comprise SEQ ID NO:12.

The tag may be a Yol tag (derived from alpha-tubulin). The Yol tag may comprise SEQ IS DO:13.

Said system for inducible expression of an adapter, wherein said inducible gene expression system is an antigen-activated inducible gene expression system, wherein said inducible promoter may be an antigen-activated inducible promoter capable of driving expression of said adapter when a cell having said inducible gene expression system may be activated Said by said antigen.

Said antigen that induces the antigen-activated inducible gene expression system! the antigen-activated inducible promoter is not the same antigen that binds to the first (poly)peptide of said adapter. Said antigen that induces the antigen antigen-activated inducible gene expression system! the antigen-activated inducible promoter may be a different antigen compared to the antigen that binds to the first (poly)peptide of said adapter.

Said system for inducible expression of an adapter, wherein said antigen that binds to the first (poly)peptide of said adapter and said antigen that induces the antigen-activated inducible promoter are different.

Said activation of the cell by the antigen that may be an antigen on a cell surface, a soluble antigen or an MHC presented antigen, may be the activation of a signaling domain of a receptor of said cell, e.g. the activation of the intracellular signaling domain of a CAR or a TCR.

Said antigen-activated inducible promoter that is activated/induced by a cell signaling pathway/cascade may be a cell surface protein promoter (e.g. CD69 promoter), a cytokine promoter (e.g. TNF promoter, IL-2 promoter), a cellular activation protein promoter (e.g. CTLA4, OX40, CD40L) or a cell surface adhesion protein promoter, or a functional part of these promoters.

The antigen-activated inducible promoter (the first nucleic acid comprising an inducible promoter) that may be operably linked to said second nucleic acid encoding said adapter may be any promoter the activation of which is responsive to a transcriptional factor that is increased when immune cells are specifically activated, such as an SP1, BATF, AP-1, IRF4, RUNX, NFAT, NF-κB, STATS or STATS sensing promoter and a minimal promoter operably linked to an inducible enhancer as described e.g. in WO2019199689A1. NFAT -inducible promoter can be exchanged with any inducible promoter that specifically binds specific transcriptional factors. Without wishing to be bound by theory, if NFAT responsive element is present, it needs a signal from TCR/CAR or other immune receptors that induces NFAT signaling.

The antigen-activated inducible promoter may comprise a minimal promoter (PMIN). Alternative minimal promoters can be used, such as minimal TATA box promoter, minimal CMV promoter or minimal IL-2 promoter. In some embodiments, the minimal promoter may be optimized for a desired level or rate of transcription.

Said system for inducible expression of an adapter, wherein said inducible gene expression system is a drug-inducible expression system and said inducible promoter is a drug-inducible promoter, wherein said inducible gene expression system further comprises a nucleic acid encoding a synthetic transcription factor for said drug-inducible promoter, wherein when a drug is administered to a cell having said inducible gene expression system, the gene expression system is induced and the adapter is expressed.

Said drug may be a synthetic drug.

Said synthetic transcription factor may comprise a DNA binding domain and drug-binding domain and an activation domain, wherein said synthetic transcription factor may be activated by binding to said drug.

Said nucleic acid encoding said synthetic transcription factor may be operatively linked to a constitutive promoter.

Constitutive promotors may be for example EF-1 alpha promoter or any other constitutive promoter that drives constitutive expression in immune cells (such as MSCV, PGK-l, UBC, CMV, CAGG, SV40 or pan-hematopoietic promoter, such as vav).

Said synthetic transcription factor may e.g. comprise a DNA-binding protein or DNA-binding domain of a transcription factor (wildtype or engineered domain e.g. zinc finger protein or POU domain), a nuclear receptor and an activation domain, and wherein said drug may be a ligand of said nuclear receptor.

Said nuclear receptor may be e.g. the estrogen receptor (ER), the progesterone (PR)-, retinoid X- or the Drosophila ecdysone receptor.

Preferentially, said synthetic transcription factor may comprise a zinc finger protein, a nuclear receptor and an activation domain, and wherein said drug may be a ligand of said nuclear receptor.

Said synthetic transcription factor may comprise a zinc finger protein, the estrogen receptor (ER) and an activation domain, and wherein said drug may be tamoxifen or a tamoxifen metabolite.

Said activation domain may be e.g. herpes virus simplex protein VP16, the tetrameric repeat of VP16's minimal activation domain VP64, derived from the p65 domain of the human endogenous transcription factor NFκB or a fusion protein comprising sequence parts of the p65 domain of the human endogenous transcription factor NFκB and sequence parts of the human heat shock factor 1.

Said tamoxifen metabolite may be endoxifen or 4-hydroxytamoxifen (4-OHT).

Said ER may be an ER having point mutations such as murine ER (G525R or G521R), human ER (G400V, M543A, L540A) or human ER (G400V, M543A, L544A).

Said drug-inducible promoter may be a hybrid promoter comprising a zinc finger binding motif and a minimal promoter that comprises a minimal promoter e.g. selected from the group consisting of E1b, TK, IL2, CMV, SV40 or any minimal TATA box promoter.

Said system for inducible expression of an adapter, wherein said synthetic transcription factor is a zinc finger protein.

Said system for inducible expression of an adapter, wherein said synthetic transcription factor is a zinc finger protein, and wherein the level of expression of adapter depends on the amount of drug administered to said cell and/or on the number of binding sites (zinc finger binding motifs) for the DNA binding of the synthetic transcription factor within the drug-inducible promoter thereby allowing a tunable control of the expression of the adapter.

Said system for inducible expression of an adapter, wherein said inducible gene expression system and said nucleic acid encoding said CAR specific for said second polypeptide of said adapter may be present in one (or in the same) immune cell.

Said system for inducible expression of an adapter, wherein said inducible gene expression system may be present in a first immune cell and said nucleic acid encoding said CAR specific for said second polypeptide of said adapter may be present in a second immune cell (in a different immune cell).

Said system for inducible expression of an adapter, wherein said inducible gene expression system is an antigen-activated inducible gene expression system as disclosed herein, and wherein said antigen-activated inducible gene expression system may be present in a first immune cell and wherein said first immune cell may comprise a CAR specific for a further antigen, wherein said CAR may comprise
i) said antigen binding domain specific for said further antigen
ii) a transmembrane domain
iii) an intracellular signaling domain, and/or

wherein said first immune cell may comprise a TCR specific for a further antigen;
and wherein said nucleic acid encoding said CAR specific for said second polypeptide of said adapter may be present in a second immune cell (in a different immune cell),

Said further antigen may be a further antigen expressed on the surface of a further target cell or on the surface of said (first) target cell or may be a soluble antigen.

Said soluble antigen may be
I) a soluble antigen of a tumor microenvironment (TME), or
II) a soluble antigen specifically associated with an autoimmune disease, or
III) a soluble antigen specifically associated with an allergic disease, or
IV) a soluble antigen specifically associated with an infectious disease, or
V) a soluble antigen specifically associated with graft rejection in a subject

Said further antigen may be a further TAA and said target cell may be said tumor cell.

Said further antigen may be expressed by a cell of the tumor microenvironment e.g. a tumor associated fibroblast.

Said further antigen may be a human infectious pathogen-derived antigen (e.g., oncoviral antigens from Epstein-Barr virus, human papillomavirus, human cytomegalovirus, or others) that is associated with tumor cells, or more generally in infectious disease (e.g., human immunodeficiency virus).

Said first immune cell and said second immune cell may be the same type of immune cells e.g. T cells (e.g. CD4 T and/or CD8 T cells) or NK cells.

Said first immune cell and said second immune cell may be different type of immune cells, e.g. the first immune cells may be a T cell and the second immune cell may be a NK cell, or said first immune cell may be e.g. a tumor infiltrating lymphocyte (TIL) and said second immune cell may be a T cell or a NK cell.

In another aspect, which is not encompassed by the wording of the claims, the present disclosure provides an immune cell comprising
a) an inducible gene expression system comprising
   I) a first nucleic acid comprising an inducible promoter operably linked to a second nucleic acid
   II) said second nucleic acid encoding an adapter comprising
      i) a first (poly)peptide, wherein said first (poly)peptide comprises an antigen binding domain that binds specifically to an antigen,
      ii) a second (poly)peptide, wherein said second (poly)peptide binds to an antigen binding domain of a chimeric antigen receptor (CAR),
b) a third nucleic acid encoding said CAR specific for said second polypeptide of said adapter, wherein said CAR comprises
   i) said antigen binding domain specific for said second (poly)peptide of said adapter
   ii) a transmembrane domain
   iii) an intracellular signaling domain.

Said adapter may be a secreted adapter.

Said adapter may be secreted by said immune cell.

Said adapter may comprise a signal peptide for secretion.

Said immune cell may be a T cell or an NK cell.

Said immune cell, wherein said inducible expression system is an antigen-activated inducible expression system, wherein said inducible promoter may be an antigen-regulated inducible promoter capable of driving expression of said adapter when a cell having said inducible gene expression system may be activated by said antigen).

Said immune cell, wherein said inducible gene expression system may be a drug-inducible expression system and said inducible promoter may be a drug-inducible promoter, wherein said inducible gene expression system further may comprise a nucleic acid encoding a synthetic transcription factor for said drug-inducible promoter, wherein when a drug may be administered to said immune cell, the gene expression system may be activated/induced and the adapter may be expressed.

Said immune cell, wherein said synthetic transcription factor may comprise a DNA binding domain and drug-binding domain and an activation domain, wherein said synthetic transcription factor may be activated by binding to said drug.

Said immune cell as disclosed herein for use in a medical treatment.

Said immune cell for use in treatment of cancer.

Said immune cell for use in treatment of a leukemia.

Said immune cell for use in treatment of a solid tumor.

Said solid tumor may be adrenal cancer, anal cancer, bile duct cancer, bladder cancer, bone cancer, brain/CNS tumors in children or adults, breast cancer, cervical cancer, colon/rectum cancer, endometrial cancer, esophagus cancer, ewing family of tumors, eye cancer, gallbladder cancer, gastrointestinal carcinoid tumors, gastrointestinal stromal tumor (GIST), gestation trophoblastic disease, hodgkin disease, kaposi sarcoma, kidney cancer, laryngeal and hypopharyngeal cancer, leukemia, acute lymphocytic leuckemia, acute myeloid leukemia, chronic lymphocytic leukemia, chronic myeloid leukemia, chronic myelomonocytic leukemia, liver cancer, lung cancer, non-small cell lung cancer, small cell lung cancer, lung carcinoid tumor, lymphoma, malignant mesothelioma, multiple myeloma, myelodysplastic syndrome, nasal cavity and paranasal sinum cancer, nasopharyngeal cancer, neuroblastoma, non-hodgkin lymphoma, oral cavity or oropharyngeal cancer, osteosarcoa, ovarian cancer, pancreatic cancer, penile cancer, pituitary tumors, prostate cancer, retinoblastoma, rhabdomyosarcoma, salivary gland cancer, skin cancer, sarcoma, basal skin cancer, squamous cell skin cancer, melanoma, merkel cell skin cancer, small intestine cancer, stomach cancer, testicular cancer, thymus cancer, thyroid cancer, uterine sarcoma, vaginal cancer, vulvar cancer, Waldenstrom macroglobulinemia, or wilms tumor.

Said immune cell for use in treatment of an infectious disease.

In another aspect, which is not encompassed by the wording of the claims, the present disclosure provides a composition comprising
a) a first immune cell comprising an inducible gene expression system comprising
   I) a first nucleic acid comprising an inducible promoter operably linked to a second nucleic acid
   II) said second nucleic acid encoding an adapter comprising
      i) a first (poly)peptide, wherein said first (poly)peptide comprises an antigen binding domain that binds specifically to an antigen,
      ii) a second (poly)peptide, wherein said second (poly)peptide binds to an antigen binding domain of a chimeric antigen receptor (CAR),
b) a second immune cell comprising a nucleic acid encoding said CAR specific for said second polypeptide of said adapter,
   wherein said CAR comprises
   i) said antigen binding domain specific for said second (poly)peptide of said adapter
   ii) a transmembrane domain
   iii) an intracellular signaling domain.

Said composition, wherein said inducible gene expression system may be a drug-inducible expression system and said inducible promoter may be a drug-inducible promoter, wherein said inducible gene expression system further may comprise a nucleic acid encoding a synthetic transcription factor for said drug-inducible promoter, wherein when a drug may be administered to said immune cell, the gene expression system may be activated/induced and the adapter may be expressed, and wherein said composition may comprise c) said drug.

Said composition as disclosed herein, wherein said synthetic transcription factor may comprise a DNA binding domain and drug-binding domain and an activation domain, wherein said synthetic transcription factor may be activated by binding to said drug.

The composition may comprise
a) a first immune cell comprising an inducible gene expression system comprising
   I) a first nucleic acid comprising an inducible promoter operably linked to a second nucleic acid
   II) said second nucleic acid encoding an adapter comprising
      i) a first (poly)peptide, wherein said first (poly)peptide comprises an antigen binding domain that binds specifically to an antigen,
      ii) a second (poly)peptide, wherein said second (poly)peptide binds to an antigen binding domain of a chimeric antigen receptor (CAR),
b) a second immune cell comprising a nucleic acid encoding said CAR specific for said second polypeptide of said adapter,
   wherein said CAR comprises
      i) said antigen binding domain specific for said second (poly)peptide of said adapter
      ii) a transmembrane domain
      iii) an intracellular signaling domain
   wherein said first immune cell may comprise a CAR specific for a further antigen, said CAR specific for said further antigen may comprise
      i) an antigen binding domain specific for said further antigen
      ii) a transmembrane domain
      iii) an intracellular signaling domain,
   wherein said inducible promoter may be an antigen-activated inducible promoter capable of driving expression of said adapter when the antigen binding domain of said CAR specific for said further antigen binds to said further antigen, thereby activating said first immune cell.

The composition may comprise
a) a first immune cell comprising an inducible gene expression system comprising
   I) a first nucleic acid comprising an inducible promoter operably linked to a second nucleic acid
   II) said second nucleic acid encoding an adapter comprising
      i) a first (poly)peptide, wherein said first (poly)peptide comprises an antigen binding domain that binds specifically to an antigen,
      ii) a second (poly)peptide, wherein said second (poly)peptide binds to an antigen binding domain of a chimeric antigen receptor (CAR),
b) a second immune cell comprising a nucleic acid encoding said CAR specific for said second polypeptide of said adapter,
   wherein said CAR comprises
      i) said antigen binding domain specific for said second (poly)peptide of said adapter
      ii) a transmembrane domain
      iii) an intracellular signaling domain
   wherein said first immune cell may comprise a TCR specific for a further antigen,
   wherein said inducible promoter may be an antigen-activated inducible promoter capable of driving expression of said adapter when said TCR specific for said further antigen binds to said further antigen, thereby activating said first immune cell.

Said compositions as disclosed herein for use in a medical treatment.

Said compositions for use in treatment of cancer.

Said compositions for use in treatment of a leukemia.

Said compositions for use in treatment of a solid tumor.

Said compositions for use in treatment of an infectious disease.

In a further aspect, which is not encompassed by the wording of the claims, the present disclosure provides a pharmaceutical composition comprising the compositions as disclosed herein, and optional a pharmaceutical acceptable carrier. Pharmaceutical acceptable carriers, diluents or excipients may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

In a further aspect, which is not encompassed by the wording of the claims, the disclsoure provides a method for treatment of a subject suffering from cancer, comprising
A) administration to said subject an immune cell comprising
   a) an inducible gene expression system comprising
      I) a first nucleic acid comprising an inducible promoter operably linked to a second nucleic acid
      II) said second nucleic acid encoding an adapter comprising
         i) a first (poly)peptide, wherein said first (poly)peptide comprises an antigen binding domain that binds specifically to an antigen,
         ii) a second (poly)peptide, wherein said second (poly)peptide binds to an antigen binding domain of a chimeric antigen receptor (CAR),
   b) a nucleic acid encoding said CAR specific for said second polypeptide of said adapter, wherein said CAR comprises
      i) said antigen binding domain specific for said second (poly)peptide of said adapter
      ii) a transmembrane domain
      iii) an intracellular signaling domain.

Said method, wherein said inducible promoter is an antigen-activated inducible promoter capable of driving expression of said adapter when said immune cell is activated by said antigen.

Said method, wherein said method comprises
B) administration of a drug to said subject,
wherein said inducible gene expression system is a drug-inducible expression system and said inducible promoter is a drug-inducible promoter, wherein said inducible gene expression system further comprises a nucleic acid encoding a synthetic transcription factor for said drug-inducible promoter , wherein when said drug is administered to said immune cell, the gene expression system is induced and the adapter is expressed.

Said method, wherein said synthetic transcription factor comprises a DNA binding domain and drug-binding domain and an activation domain, wherein said synthetic transcription factor is activated by binding to said drug.

Said method, wherein said drug may be administered simultaneously with, before or after the administration of said immune cell.

In another aspect, which is not encompassed by the wording of the claims, the disclsoure provides a method for treatment of a subject suffering from cancer, comprising
A) administration to said subject a first immune cell comprising
   a) an inducible gene expression system comprising
      I) a first nucleic acid comprising an inducible promoter operably linked to a second nucleic acid
      II) said second nucleic acid encoding an adapter comprising
         i) a first (poly)peptide, wherein said first (poly)peptide comprises an antigen binding domain that binds specifically to an antigen,
         ii) a second (poly)peptide, wherein said second (poly)peptide binds to an antigen binding domain of a chimeric antigen receptor (CAR),
B) administration to said subject a second immune cell comprising a nucleic acid encoding said CAR specific for said second polypeptide of said adapter,
   wherein said CAR comprises
   i) said antigen binding domain specific for said second (poly)peptide of said adapter
   ii) a transmembrane domain
   iii) an intracellular signaling domain.

Said method, wherein said method comprises
C) administration of a drug to said subject,
wherein said inducible gene expression system is a drug-inducible expression system and said inducible promoter is a drug-inducible promoter, wherein said inducible gene expression system further comprises a nucleic acid encoding a synthetic transcription factor for said drug-inducible promoter , wherein when said drug is administered to said immune cell, the gene expression system is induced and the adapter is expressed.

Said method, wherein said synthetic transcription factor comprises a DNA binding domain and drug-binding domain and an activation domain, wherein said synthetic transcription factor is activated by binding to said drug.

Said method, wherein said drug may be administered simultaneously with, before or after the administration of said immune cells.

Said method, wherein said inducible promoter is capable of driving expression of said adapter when said first immune cell is activated.

### Definitions

Unless defined otherwise, technical and scientific terms used herein have the same meaning as commonly understood by one of ordinary skill in the art to which this invention belongs. As used herein the term "comprising" or "comprises" is used in reference to compositions, methods, and respective component(s) thereof, that are essential to the method or composition, yet open to the inclusion of unspecified elements, whether essential or not.

The term "system for inducible expression of an adapter in immune cells" refer to a combination of nucleic acids in a one-cell constellation or two-cell constellation as disclosed herein. In this context, the term "system" may be used interchangeable with "a combination of nucleic acids" or "a composition of nucleic acids".

In general, a CAR may comprise an extracellular domain (extracellular part) comprising the antigen binding domain, a transmembrane domain and a cytoplasmic signaling domain (intracellular signaling domain). The extracellular domain may be linked to the transmembrane domain by a linker or spacer. The extracellular domain may also comprise a signal peptide. In some embodiments of the invention the antigen binding domain of a CAR binds a tag that is coupled to a (poly)peptide ("tagged" polypeptide or adapter comprising said first (polypeptide) as disclosed herein and said second (poly)peptide as disclosed herein), wherein the tagged polypeptide may bind to a disease-associated antigen such as a tumor associated antigen (TAA) that may be expressed on the surface of a cancer cell or an antigen expressed by cells of the tumor microenvironment or an infection-associated antigen that may be expressed by a pathogen-infected cell

Said second (poly)peptide of the adapter that may bind to an antigen binding domain of a chimeric antigen receptor (CAR) as disclosed herein may also be referred to as a "tag" (of a tagged polypeptide) and said CAR specific for said second polypeptide of said adapter may also be referred to as anti-tag CAR or adapterCAR (adCAR) or "universal CAR".

Such an anti-tag CAR is disclosed e.g. in US9233125B2.

Generally, the tags of an anti-tag CAR may be coupled directly or indirectly to a polypeptide (the tagged polypeptide), wherein the polypeptide may bind to said disease associated antigen expressed on the (cell) surface of a target. Often the tag may be e.g. dextran or a hapten such as biotin or fluorescein isothiocyanate (FITC) or phycoerythrin (PE) or thiamin. But the tag as part of the adapter of the present invention regularly may be a (poly)peptide (the second (poly)peptide of the adapter as disclosed herein) that may be generated in the immune cell via transcription and translation of an exogeneous nucleic acid sequence introduced into said immune cell.

The tag may be a (poly)peptide comprising at least 4 amino acids

The tag may be a (polypeptide) comprising at least 4 amino acids and not more than 8, 10, 15, 20, 25, 30, 40, 50, 60, 70, 80, 90, 100 or 200 amino acids.

The tag may have high proteolytic stability and low immunogenicity in humans relative to (poly)peptides in general.

The tag may be located at or near the N terminus or the C terminus of said first polypeptide. The tag may further be integrated in the sequence of said first polypeptide, thus not located at or near the N terminus or the C terminus of said first polypeptide.

The tag may comprise an epitope of a protein.

The tag may comprise an epitope of a protein naturally occurring in a subject or in a circulatory system of a subject.

The tag may be e.g. a hormone, a cytokine, a chemokine, a growth factor, a cell adhesion molecule, a signaling peptide, a receptor, a cell surface peptide or fragments thereof. The tag may be a ligand or a fragment thereof. The ligand may be a hormonal ligand. The ligand may be a peptide ligand. The tag may be an antigen, an epitope, including linear and nonlinear epitopes. The tag may be a tumor associated antigen. Alternatively, the tag may not be a tumor associated antigen.

The tag may comprise an epitope of a neo-antigen, wherein said epitope comprises the mutation of the antigen.

The tag may comprise an epitope of a neo-antigen as may occur in tumorigenesis, wherein said epitope comprises the mutation of the antigen.

The tag may comprise an epitope of a neo-antigen, wherein said epitope comprises the mutation of the antigen, wherein said antigen may be a protein naturally occurring in a subject or in a circulatory system of a subject.

The tag may be a (poly)peptide that does not naturally occur in a subject.

The tag may be a (poly)peptide that does not naturally occur in the circulatory system of a subject.

The tag may be a (poly)peptide that does not naturally occur in the circulatory system of a healthy subject.

The tag may be e.g. one of the following peptides: c-Myc-tag, Strep-Tag II, Flag-Tag, Polyhistidine-tag, Avi-Tag, Calmodulin binding protein-Tag, Yol-tag (derived from alpha-tubulin), E-tag, HA-Tag, S-tag, SBP-tag or V5 tag.

A "signal peptide" refers to a peptide sequence that directs the transport and localization of the protein within a cell, e.g. to a certain cell organelle (such as the endoplasmic reticulum) and/or the cell surface.

Generally, an "antigen binding domain" refers to the region of the CAR that specifically binds to an antigen, e.g. to a tumor associated antigen (TAA) or tumor specific antigen (TSA). More specifically the "antigen binding domain" of an anti-tag CAR specifically may bind to a tag present on a tagged polypeptide, wherein the polypeptide may bind to said disease associated antigen expressed on the (cell) surface of a target. The CARs may comprise one or more antigen binding domains (e.g. a tandem CAR). Generally, the targeting regions on the CAR are extracellular. The antigen binding domain may comprise an antibody or an antigen binding fragment thereof. The antigen binding domain may comprise, for example, full length heavy chain, Fab fragments, single chain Fv (scFv) fragments, divalent single chain antibodies or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G₄/S)₃-linker".

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art.

"Spacer" or "hinge" as used herein refers to the hydrophilic region which is between the antigen binding domain and the transmembrane domain. The CARs of the invention may comprise an extracellular spacer domain but is it also possible to leave out such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. The splitting key signaling and antigen recognition modules enable for a small molecule-dependent, titratable and reversible control over CAR cell expression (e.g. WO2014127261A1) due to small molecule-dependent heterodimerizing domains in each polypeptide of the CAR.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR, otherwise it is indicated explicitly as an inhibitory CAR (iCAR)). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) and co-receptors that initiate signal transduction following antigen receptor engagement.

Generally, T cell activation can be mediated by two distinct classes of cytoplasmic signaling sequences, firstly those that initiate antigen-dependent primary activation through the TCR (primary cytoplasmic signaling sequences, primary cytoplasmic signaling domain) and secondly those that act in an antigen-independent manner to provide a secondary or costimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs).

Examples of ITAM containing primary cytoplasmic signaling domains often used in CARs are that those derived from TCRζ (CD3ζ), FcRgamma, FcRbeta, CD3gamma, CD3delta, CD3epsilon, CD5, CD22, CD79a, CD79b, and CD66d. Most prominent is sequence derived from CD3ζ.

The cytoplasmic domain of the CAR may be designed to comprise the CD3ζ signaling domain by itself or combined with any other desired cytoplasmic domain(s). The cytoplasmic domain of the CAR can comprise a CD3ζ chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, PD-1, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3ζ and the signaling domain of CD28. In another example the cytoplasmic domain may comprise the signaling domain of CD3ζ and the signaling domain of CD137. In a further example, the cytoplasmic domain may comprise the signaling domain of CD3ζ, the signaling domain of CD28, and the signaling domain of CD137.

As aforementioned either the extracellular part or the transmembrane domain or the cytoplasmic domain of a CAR may also comprise a heterodimerizing domain for the aim of splitting key signaling and antigen recognition modules of the CAR.

The CAR may be further modified to include on the level of the nucleic acid encoding the CAR one or more operative elements to eliminate CAR expressing immune cells by virtue of a suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent is administered to a cell being transduced with said inducible gene expression system, the gene expression system is induced and said CAR is expressed on the surface of said transduced cell.

In some embodiments, the endodomain may contain a primary cytoplasmic signaling domain or a co-stimulatory region, but not both.

In some embodiment of the invention the CAR may be a "SUPRA" (split, universal, and programmable) CAR, where a "zipCAR" domain may link an intra-cellular costimulatory domain and an extracellular leucine zipper (WO2017/091546). This zipper may be targeted with a complementary zipper fused e.g. to an scFv region to render the SUPRA CAR T cell tumor specific. This approach would be particularly useful for generating universal CAR T cells for various tumors; adapter molecules could be designed for tumor specificity and would provide options for altering specificity post-adoptive transfer, key for situations of selection pressure and antigen escape.

The CARs of the present invention may be designed to comprise any portion or part of the above-mentioned domains as described herein in any order and/or combination resulting in a functional CAR, i.e. a CAR that mediated an immune effector response of the immune effector cell that expresses the CAR as disclosed herein.

The term "tagged polypeptide" as used herein refers to a polypeptide that has bound thereto directly or indirectly at least one additional component, i.e. the tag. The tagged polypeptide as used herein is able to bind an antigen expressed on a target cell. The polypeptide may be an antibody or antigen binding fragment thereof that binds to an antigen expressed on the surface of a target cell such as a tumor associated antigen on a cancer cell. The polypeptide of the tagged polypeptide alternatively may be a cytokine or a growth factor or another soluble polypeptide that is capable of binding to an antigen of a target cell. Said polypeptide of the tagged polypeptide alternatively may be an affibody or a ligand binding domain from a naturally occurring receptor.

As used herein, a "T cell receptor" or "TCR" refers to the antigen-recognition molecules present on the surface of T-cells. During normal T-cell development, each of the four TCR genes, α, β, γ, δ can rearrange leading to highly diverse TCR proteins.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibodyimmunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), single domain antibodies (nanobodies), diabodies, dsFv, Fab', diabodies, single-chain antibody molecules, and multispecific antibodies formed from antibody fragments. The antibody or antibody fragment may be human, fully human, humanized, human engineered, non-human, and/or chimeric. The non-human antibody or antibody fragment may be humanized to reduce immunogenicity to humans, while retaining the specificity and affinity of the parental non-human antibody. Chimeric antibodies may refer to antibodies created through the joining of two or more antibody genes which originally encoded for separate antibodies.

The terms "having specificity for", "specifically binds" or "specific for" with respect to an antigen-binding domain of an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain is specific. An antigen-binding domain that specifically binds to an antigen may bind also to different allelic forms of the antigen (allelic variants, splice variants, isoforms etc.). This cross reactivity is not contrary to the definition of that antigen-binding domain is specific.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid. The term "antigen" as used herein refers to a molecular entity that may be expressed e.g. on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The term "soluble antigen" as used herein refers to an antigen that is not immobilized on surfaces such as beads or cell membranes.

The terms "immune cell" or "immune effector cell" may be used interchangeably and refer to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, regulatory T cells (Treg), monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells are T cells and NK cells. Tumor infiltrating lymphocytes (TILs) are T cells that have moved from the blood of a subject into a tumor. These TILs may be removed from a patient's tumor by methods well known in the art, e.g. enzymatic and mechanic tumor disruption followed by density centrifugation and/or cell marker specific enrichment. TILs are genetically engineered as disclosed herein, and then given back to the patient. "Effector function" means a specialized function of a cell, e.g. in a T cell an effector function may be cytolytic activity or helper activity including the secretion of cytokines.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based or NK cell-based cytotoxic responses to attack cancer cells. T cells that have a natural or genetically engineered reactivity to a patient's cancer are generated *in-vitro* and then transferred back into the cancer patient. Then the immunotherapy is referred to as "CAR cell immunotherapy" or in case of use of T cells only as "CAR T cell therapy" or "CAR T cell immunotherapy".

The term "treatment" as used herein means to reduce the frequency or severity of at least one sign or symptom of a disease.

The term "autologous" as used herein refers to any material derived from the same subject to who it is later re-introduced.

The term "allogeneic" as used herein refers to any material derived from a different subject of the same species as the subject to who the material is re-introduced.

The terms "therapeutically effective amount" or "therapeutically effective population" mean an amount of a cell population which provides a therapeutic benefit in a subject.

As used herein, the term "subject" refers to an animal. Preferentially, the subject is a mammal such as mouse, rat, cow, pig, goat, chicken dog, monkey or human. More preferentially, the subject is a human. The subject may be a subject suffering from a disease such as cancer (a patient) or from an autoimmune disease or from a allergic disease or from an infectious disease or from graft rejection.

The term "expression" as used herein is defined as the transcription and/or translation of a particular nucleotide sequence driven by its promoter in a cell.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially T cells can be manipulated by recombinant methods well known in the art to express stably or transiently peptides or proteins which are not expressed in these cells in the natural state. For example, T cells, preferentially human T cells are engineered to express an artificial construct such as a chimeric antigen receptor on their cell surface.

The term "cancer" is known medically as a malignant neoplasm. Cancer is a broad group of diseases involving unregulated cell growth and includes all kinds of leukemia. In cancer, cells (cancerous cells) divide and grow uncontrollably, forming malignant tumors, and invading nearby parts of the body. The cancer may also spread to more distant parts of the body through the lymphatic system or bloodstream. There are over 200 different known cancers that affect humans.

The terms "nucleic acid" or "polynucleotide" as used interchangeably herein refer to polymers of nucleotides. Polynucleotides, which can be hydrolyzed into monomeric "nucleotides." The monomeric nucleotides can be hydrolyzed into nucleosides. As used herein, the term "polynucleotides" encompasses, but is not limited to, all nucleic acid sequences which are obtained by any means available in the art, including, without limitation, recombinant means, i.e., the cloning of nucleic acid sequences from a recombinant library or a cell genome, using ordinary cloning technology and PCR, and the like, and by synthetic means.

Peptides are short chains of between two and fifty amino acids, linked by peptide bonds. Chains of less than ten or fifteen amino acids may also be called oligopeptides.

A polypeptide is a longer, continuous, unbranched peptide chain of up to fifty amino acids or more. A polypeptide that contains more than fifty amino acids may also called a protein.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

As used herein, the terms "promoter" or "regulatory sequence" mean a nucleic acid sequence which is required for transcription of a gene product operably linked to the promoter/regulatory sequence. In some instances, this sequence may be the core promoter sequence and in other instances, this sequence may also include an enhancer sequence and other regulatory elements which are required for transcription of the gene product. The promoter/regulatory sequence may, for example, be one which expresses the gene product in a tissue-specific manner.

The term "minimal promoter (PMIN)" as used herein refers to the smallest genetic element that is able to induce transcription of a gene located downstream of said minimal promoter. Eukaryotic promoters of protein-coding genes have one or more of three conserved sequences in this region (i.e. the TATA-box, initiator region, and downstream promoter element). A minimal promoter enables low basal leakiness in the absence of specific transcriptional activators and high expression when transcription activators are bound upstream of minimal promoter at their specific DNA binding sites. Alternative minimal promoters can be used, such as minimal TATA box promoter, minimal CMV promoter or minimal IL-2 promoter.

The minimal promoter may be engineered / modified by the introduction of binding sites for specific transcription factors (e.g. required for the drug-inducible system, but also for the antigen-activated inducible system the promoter may contain several repeats e.g. of the NFAT binding site).

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only in the presence or absence of certain conditions such as, for example, when an inducer ( e.g. an induction signal, or an induction agent such as a drug, metal ions, alcohol, oxygen, etc.) is present in the cell. The inducer may be e.g. the activation of the intracellular signaling domain of a CAR.

Constitutive promotors that are operatively linked to a transgene may be for example EF-1 alpha promoter or any other constitutive promoter that drives constitutive expression in immune cells (such as MSCV, PGK-1, UBC, CMV, CAGG, SV40 or pan-hematopoietic promoter, such as vav).

The inducible promoter that is operably linked to the polynucleotide encoding e.g. the adapter as disclosed herein may be any promoter the activation of which is responsive to a transcriptional factor that is increased when immune cells are specifically activated or localized to a given microenvironment (could be e.g. a tumor microenvironment), such as an SP1, BATF, AP-1, IRF4, RUNX, NFAT, NF-κB, STATS or STATS sensing promoter and a minimal promoter operably linked to an inducible enhancer as described e.g. in WO2019199689A1. The inducible promoter further may comprise a minimal promoter operably linked to an effector. NFAT -inducible promoter can be exchanged with any inducible promoter that specifically binds specific transcriptional factors. Without wishing to be bound by theory, if NFAT responsive element is present, it needs a signal from TCR/CAR or other immune receptors that induces NFAT signaling.

The inducible promoter may comprise a minimal promoter (PMIN). Alternative minimal promoters can be used, such as minimal TATA box promoter, minimal CMV promoter or minimal IL-2 promoter. In some embodiments, the minimal promoter may be optimized for a desired level or rate of transcription.

In a specific variant, the inducible promoter may be a drug-inducible promoter.

Such a system may comprise a nucleic acid comprising a promoter inducible by a drug, e.g. by a synthetic drug. By utilizing a drug-inducible promoter, a transgene expression can be turned on and off in order to avoid toxic side effects and/or to allow the cells to rest during remission. Many of these systems use chimeric transcriptional regulators (e.g. synthetic transcription factors)

In one variant the inducible promoter may be inducible by a drug, i.e. a drug-inducible promoter. The drug is selected based on safety record, favorable pharmacokinetic profile, tissue distribution, a low partition coefficient between the extracellular space and cytosol, low immunogenicity, low toxicities, and/or high expression in lymphocytes. In some alternatives, the inducible promoter is activated by a transcriptional activator (e.g. a synthetic transcription factor) that interacts with a drug. The transcriptional activator is activated or able to bind to and activate the inducible promoter in the presence of the drug. A specific alternative of a drug is a drug that binds to an estrogen receptor ligand binding domain of a transcriptional activator. In some alternatives, the drug includes tamoxifen, its metabolites, analogs, and pharmaceutically acceptable salts and/or hydrates or solvates thereof.

The term "synthetic transcription factor" as used herein may comprise a DNA-binding domain, a drug inducible domain (a drug binding domain) and an effector (activation) domain, that are linked and/or fused whereby the individual domains can be arranged in any order.

A DNA binding domain of a synthetic transcription factor may be a protein or a portion of a protein that specifically recognize the DNA binding motif of the drug-inducible promoter and mediate the binding of the synthetic transcription factor to this DNA sequence. Besides zinc finger proteins, TALE (transcription activator-like effector) and Cas9 (Clustered Regulatory Interspaced Short Palindromic Repeats -associated system) may be engineered to recognize a specific DNA sequence. Moreover, the DNA binding domain of naturally occurring transcription factors (e.g. POU homeodomain) may be employed.

Said DNA binding domain may be e.g. a zinc finger protein (or the DNA binding domain thereof) or a protein comprising or consisting of a POU domain.

DNA binding motifs of drug-inducible promoters are specific DNA sequences that are directly or indirectly (in case of Cas9) recognized by the DNA-binding domain of the synthetic transcription factor. E.g. each zinc finger domain specifically recognizes a DNA sequence of 3 bp, thus a three-finger zinc finger protein can be designed to recognize a 9 bp sequence.

Drug-binding domain of a synthetic transcription factor refers to a protein or a portion of a protein that binds to a drug or a ligand of the domain. Upon drug binding, the drug-binding domain enables the transition from an inactive to an active synthetic transcription factor. This transition may include the release of inactivation factors and/or the translocation of the synthetic transcription factor from the cytoplasm to the nucleus. Examples of drug binding domains are nuclear receptors, extracellular domains of receptors, antigen/substance binding proteins (also dimerizers) and/or active sites of enzymes.

An activation domain of a synthetic transcription factor refers to a protein or a portion of a protein that autonomously facilitates the recruitment of the transcriptional machinery to initiate mRNA transcription. Examples of activation domains are VP16, VP64, fragments of NFkB p65, heat shock factor 1 and combinations thereof.

E.g. the synthetic transcription factor may comprise a zinc finger protein, the estrogen receptor (ER) and an activation domain, and wherein said drug may be tamoxifen or a tamoxifen metabolite. Said activation domain may be e.g. herpes virus simplex protein VP16, the tetrameric repeat of VP16's minimal activation domain VP64, parts of the p65 domain of the human endogenous transcription factor NFκB or a fusion protein comprising fragments of human NFκB p65 and heat shock factor 1. Said tamoxifen metabolite may be endoxifen or 4-OHT. Said ER may be a ER having point mutations such as murine ER (G525R) or (G521R), human ER (G400V, M543A, L540A) or human ER (G400V, M543A, L544A).

The drug-inducible promoter may be a hybrid promoter comprising a DNA binding motif for said DNA binding domain of the synthetic transcription factor and a minimal promoter.

Said drug-inducible promoter may be a hybrid promoter comprising a zinc finger binding motif and a minimal promoter that comprises a minimal promoter selected from the group consisting of E1b, TK, IL2, CMV, SV40.

The term "inducible (gene) expression system" refers to the expression of an exogenous polypeptide (a transgene), herein normally the adapter as disclosed herein in an immune cell. The inducible (gene) expression system may be an "antigen-activated inducible gene expression system", i.e. the inducible expression system may be activated in a cell having said inducible gene expression system, when an antigen/ligand is bound by directly or upon MHC presentation by a receptor of the cell such as a CAR or an TCR. Said binding of the antigen/ligand to the receptor may induce a signal cascade within the cell that subsequently may lead to the induction of the expression of the introduced gene (or transgene), herein normally the adapter. Said antigen/ligand that may induce a signal cascade within the cell, when bound to the cognate receptor of the cell may also be referred to as "inducing signal".

Alternatively, the inducible gene expression system may be a drug-inducible gene expression system, i.e. the inducible gene expression system may be activated in a cell having said inducible gene expression system, when a drug, e.g. a synthetic drug such as tamoxifen may be introduced to the cell. Said drug in the cell may bind to a synthetic transcription factor and subsequently may lead to the induction of the expression of the transgene, herein the adapter.

Said drug may also be referred to as "inducing agent".

Both kinds of inducible gene expression systems may be used in a one cell and/or two cell system as disclosed herein.

In the presence of an induction signal or an induction agent, the inducible expression system drives expression of the exogenous polypeptide. In an induced system, withdrawal of the induction signal or the induction agent may reduce and/or halt expression of the exogenous polypeptide. Upon re-introduction of the induction signal or the induction agent, the system can then be re-induced and restart the expression of the exogenous polypeptide, i.e. the adapter. The inducible (gene) expression system may be inducible by an induction signal, e.g. by the activation of the intracellular signaling domain of a CAR or a TCR by binding of an antigen/ligand as disclosed herein ("activation"-induced) or by an induction agent, such as a (synthetic) drug as disclosed herein ("drug-induced").

In some embodiments, an inducible (gene) expression system as disclosed herein may also provide tunable control of the expression of the adapter. As used herein, the term "tunable control" refers to the ability to control the expression level of the adapter as disclosed herein. For example, the level of induced expression of an adapter may depend on the amount of induction agent or induction signal that is present. For example, the presence of a higher amount of induction agent, e.g. a synthetic drug may induce higher levels of expression of an adapter as compared to the presence of a lower amount of induction agent. As such, the inducible or tunable expression of an adapter may be dose-dependent with respect to the amount of induction agent present.

Besides the inducer drug dose, in some embodiments, an inducible (gene) expression system as disclosed herein may also provide tunable control of the expression of the adapter by the number of response elements for the synthetic transcription factor. As used herein, the term "tunable control" refers to the ability to control the expression level of the adapter as disclosed herein. For example, the level of induced expression of an adapter may depend on the number of response elements in other words the number of binding sites for the synthetic transcription factor within the inducible promoter. For example, upon binding of five synthetic transcription factor molecules to an inducible promoter comprising five binding sites a transcriptional output i.e. a higher level of expression of an adapter is induced as compared to constructs comprising two response elements within the inducible promoter. As such, the inducible or tunable expression of an adapter may be dependent from the number of response elements for the synthetic transcription factor.

### Disclsoure

The immune cells expressing the adapterCAR and the inducible adapter in the same cell as disclosed herein may be for use in treatment of cancer in a subject suffering from cancer. The adapter may be specifically bound by the adapterCAR and the adapter specifically may bind to an antigen of said cancer. Immune cells, e.g. T cells or NK cells of a subject are isolated. The subject may suffer from said cancer or may be a healthy subject. These cells are genetically modified *in vitro* or *in vivo* to express said CAR and in an inducible manner said adapter. These engineered cells may be activated and expanded *in vitro* or *in vivo.* In a cellular therapy these engineered cells are infused to a recipient in need thereof. These cells may be a pharmaceutical composition (said cell plus pharmaceutical acceptable carrier). The infused cells are able to kill (or at least stop growth of) cancerous cells in the recipient, when the expression of the adapter has been induced. Induction of the expression may be triggered by applying a synthetic drug such as tamoxifen, when the inducible expression system is a drug-inducible system as disclosed herein. The recipient may be the same subject from which the cells was obtained (autologous cell therapy) or may be from another subject of the same species.

The immune cells, preferentially T cells or NK cells engineered to express said CAR and said adapter may be administered either alone, or as a pharmaceutical composition in combination with diluents and/or with other components such as IL-2 or other cytokines or cell populations. Briefly, pharmaceutical compositions of the present invention may comprise a cell population of genetically modified cells as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Preferentially, the compositions are formulated for intravenous administration. The administration of cell compositions to the subject may be carried out in any convenient manner known in the art.

Pharmaceutical compositions may be administered in a manner appropriate to the disease to be treated. Appropriate dosages may be determined by clinical trials. But the quantity and frequency of administration will also be determined and influenced by such factors as the condition of the patient, and the type and severity of the patient's disease.

A pharmaceutical composition comprising the immune cells, preferentially T cells or NK cells as disclosed herein may be administered at a dosage of 10⁴ to 10⁹ cells/kg body weight, preferably 10⁵ to 10⁶ cells/kg body weight. The cell compositions may also be administered several times at these dosages. The compositions of cells may be injected directly into the blood stream, a tumor, lymph node, or site of infection.

The drug for inducing the expression of the adapter may be administered in a manner appropriate to the disease to be treated. Appropriate dosages may be determined by clinical trials. But the quantity and frequency of administration will also be determined and influenced by such factors as the condition of the patient, and the type and severity of the patient's disease. The drug may be injected directly into the blood stream, applied to the skin or taken orally. The drug may be administered several times also at variable dosages. The drug will be formulated according to the administration route.

The cells may be activated and expanded to therapeutic effective amounts using methods known in the art.

The cells may be used in combination with e.g. chemotherapy, radiation, immunosuppressive agents, antibodies or antibody therapies.

A composition of immune cells as disclosed herein may be for use in treatment of cancer in a subject suffering from cancer or may be for use in treatment of a viral infection in a patient. The composition may comprise immune cells expressing an inducible adapter as disclosed herein, the immune cells may be e.g. TILs, and the composition may comprise immune cells expressing the adapterCAR as disclosed herein. The adapter may be specifically bound by the adapterCAR and the adapter specifically may bind to an antigen of said cancer or to the antigen of a pathogen associated with the viral infection. Immune cells, e.g. T cells, TILs or NK cells of a subject are isolated. The immune cells may be further isolated on the basis of TCR reactivity to oncoviral antigens or tumor neo-antigens (e.g., isolation of cytomegalovirus-reactive T cells via PepTivator^{™} CMV p65 peptide activation (Miltenyi Biotec) and isolation of activated T cells via the CliniMACs^{™} Cytokine Capture System (Miltenyi Biotec)). The subject may suffer from said cancer or may be a healthy subject. These cells are genetically modified *in vitro* or *in vivo* to express said CAR and in an inducible manner said adapter in two different cells. These engineered cells may be activated and expanded *in vitro* or *in vivo.* In a cellular therapy these engineered cells are infused to a recipient in need thereof. These cells may be a pharmaceutical composition (said cell plus pharmaceutical acceptable carrier). The infused immune cells expressing the CAR are able to kill (or at least stop growth of) cancerous cells in the recipient, when the expression of the adapter has been induced. Induction of the expression may be triggered by applying a synthetic drug such as tamoxifen, when the inducible expression system is a drug-inducible system as disclosed herein. The recipient may be the same subject from which the cells was obtained (autologous cell therapy) or may be from another subject of the same species.

The immune cells, preferentially T cells, TILs or NK cells engineered to express said CAR and said adapter in two different cells may be administered either alone, or as a pharmaceutical composition in combination with diluents and/or with other components such as IL-2 or other cytokines or cell populations. Briefly, pharmaceutical compositions of the present invention may comprise a cell population of genetically modified cells as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Preferentially, the compositions are formulated for intravenous administration. The administration of cell compositions to the subject may be carried out in any convenient manner known in the art.

Pharmaceutical compositions may be administered in a manner appropriate to the disease to be treated. Appropriate dosages may be determined by clinical trials. But the quantity and frequency of administration will also be determined and influenced by such factors as the condition of the patient, and the type and severity of the patient's disease. A pharmaceutical composition comprising the immune cells, preferentially T cells, TILs or NK cells as disclosed herein may be administered at a dosage of 10⁴ to 10⁹ cells/kg body weight, preferably 10⁵ to 10⁶ cells/kg body weight. The cell compositions may also be administered several times at these dosages. The compositions of cells may be injected directly into a tumor, lymph node, or site of infection.

The drug for inducing the expression of the adapter may be administered in a manner appropriate to the disease to be treated. Appropriate dosages may be determined by clinical trials. But the quantity and frequency of administration will also be determined and influenced by such factors as the condition of the patient, and the type and severity of the patient's disease. The drug may be injected directly into the blood stream, applied to the skin or taken orally. The drug may be administered several times also at variable dosages. The drug will be formulated according to the administration route. The cells may be activated and expanded to therapeutic effective amounts using methods known in the art.

The cells may be used in combination with e.g. chemotherapy, radiation, immunosuppressive agents, antibodies or antibody therapies.

The immune cells expressing the CAR and the immune cells expressing the inducible adapter may be administered simultaneously, or the immune cells expressing the CAR may be administered before the immune cells that express the inducible adapter, or vice versa. The drug for inducing the expression of the adapter may be administered simultaneously with the immune cells to the patient, or after the administration of the immune cells.

The immune cells expressing the inducible adapter as disclosed herein may be TILs that traffic to the (solid) tumor. They may function as vehicle for in situ delivery of the adapter.

The immune cells may be modified to express an "activation-inducible" or "drug-inducible" adapter that targets human pathogen-infected cells (e.g, HIV-1 infected cells). These cells may be isolated e.g. on the basis of their TCR reactivity to pathogen-associated antigens or tumor neoantigens, through the use of pathogen-associated peptide pools to activate T cells, followed e.g. by isolation of activated T cells via the CliniMACs^{™} Cytokine Capture System (Miltenyi Biotec), or other isolation modalities. These immune cells, upon trafficking to the site of infection and TCR recognition of pathogen-infected cells, may drive expression of the adapter specific for the antigen of the pathogen through an "activation-inducible" promoter. Alternatively, drug-inducible expression of the adapter may be used to controllably produce the adapter specific for the antigen of the pathogen, specifically at the site of infection. These engineered immune cells may also comprise the adapterCAR specific for said adapter that binds to the antigen of the pathogen, or other engineered immune cells may comprise the CAR that do not express the adapter.

Immune cells may be modified with an activation-inducible cassette (referred to as activation-inducible cells), whereby a promoter responsive to TCR/CAR-mediated signaling drives expression of an adapter molecule. These cells can be selected on the basis of their reactivity to tumor antigens by isolating cells from specific areas of the body (e.g., TILs isolated from the tumor site), or by selecting T cells with TCRs reactive against oncoviral antigens (e.g., CMV PepTivator-activated T cells), or against tumor neoantigens (e.g., tumor neoantigen peptide pool-activated T cells). Alternatively, T cells can be transduced with a CAR or TCR specific for any of the above-mentioned antigen categories. Upon TCR/CAR recognition of the TAA, oncoviral, or tumor neo-antigen, the TCR-reactive promoter drives expression of an anti-tumor adapter molecule. These tagged adapter molecules are specific for a tumor antigen. The tag moiety (in this case: 6xHis Tag) of the adapter molecule is recognized by the extracellular recognition domain of the adapter CAR (in this case: anti-His₆ specific CAR), which is expressed by a second immune cell. The tagged adapter molecule constitutes a bridging molecule redirecting the adapter CAR cells to the tumor. Consequently, tumor cells are lysed by the adapter CAR cell.

Immune cells may be modified with a drug-inducible expression cassette (referred to as drug-inducible cells). In the "off' state those inducible cells cannot be distinguished from unmodified immune cells. Upon the administration of the inducer drug, the inducible expression cassette is activated and tagged adapter molecules are transcribed and secreted by the immune cell. These tagged adapter molecules are specific for a tumor antigen. The tag moiety (in this case: 6xHis Tag) of the adapter molecule is recognized by the extracellular recognition domain of the adapter CAR (in this case: anti-His₆ specific CAR), which is expressed by a second immune cell. The tagged adapter molecule constitutes a bridging molecule redirecting the adapter CAR cells to the tumor. Consequently, tumor cells are lysed by the adapter CAR cell.

### Examples

### Example 1: Generation of anti-tag CAR T cells and drug-inducible T cells

### 1.1 Construct design

Adapter CAR T cells comprise an anti-His₆ scFv as binding moiety. The scFv is linked to an human CD8 transmembrane domain via an hIgG4 hinge domain. The signaling domain is composed of 4-1BB, CD28 and CD3ζ. A furin P2A site followed by a truncated LNGFR is 3' of the CAR construct. The LNGFR is used as a transduction marker.

Inducible T cells constitutively express a synthetic transcription factor via the PGK promoter. The synthetic transcription factor is composed of a 3-finger zinc finger protein, referred to as N1, a murine estrogen receptor (G525R) and the activation domain VP64. The sequence of the synthetic transcription factor is linked via a furin P2A site to LNGFR, which was used as transduction marker. The inducible gene expression cassette also comprises the sequence for the tagged adapter molecule (in this case: a His₆-tagged anti-CD 19 Fab) whereby the transcription of the tagged adapter molecule is regulated by the inducible promoter. The inducible promoter is composed of the binding site for the N1 zinc finger (five repeats) linked to an E1b minimal promoter.

### 1.2 Generation of LV particles and titration

Lentiviral vector particles were manufactured via transient transfection of HEK-293T cells. The lentiviral vector particles were pseudotyped with VSV-G. For transfection HEK-293T cells were seeded in T175 culture flasks in DMEM (Biowest) supplemented with 2 mM L-Glutamine (Lonza) and 10 % FCS (Biochrom) 3 days prior to transfection. At the day of transfection the culture medium was removed and replaced by DMEM (Biowest) supplemented with 2 mM L-Glutamine (Lonza). The cells were transfected with a three plasmid system encoding for VSV-G, gag/pol/rev and the psi positive transfer vector (anti-tag CAR or inducible cassette). After 48h the supernatant was collected and centrifuged for 10 min at 1000 rpm to remove cellular debris. In addition, the supernatant was filtrated trough a 0.45 µm filter. The pellet was re-suspended in ice cold PBS and stored at - 80 °C.

A functional titer of VSV-G pseudotyped lentiviral vector particles was determined via titration on Sup-T1 cells. 2E5 cells were seeded in 100 µL RPMI (Biowest) supplemented with 2 mM L-Glutamine (Lonza) in 96 well round bottom plates. For transduction 100 µL of serial diluted lentiviral vector particles were added to the seeded cells. 90 µL RPMI (Biowest) supplemented with 2 mM L-Glutamine (Lonza) and 10 % FCS (Biochrom) was added after 24 h. The frequency of transduced cells was quantified after 96 h by flow cytometry using a LNGFR APC conjugate (Miltenyi Biotec). Based on the frequency of LNGFR positive cells, the number of seeded cells and the volume of lentiviral particle used for transduction, the titer was calculated. The titer was expressed in transducing units per mL.

### 1.3 Transduction, cultivation and analysis of anti-tag CAR T cells and inducible T cells

Anti-tag CAR T cells and inducible T cells were manufactured using primary T cells from healthy donors. T cells were isolated from PBMC with the PAN T cell isolation Kit (Miltenyi Biotec) according to the manufactures protocol. Prior to transduction 2E6 T cells were seeded in a 24 well plate with 2 mL TexMACS medium (Miltenyi Biotec) supplemented with IL-7 (Miltenyi Biotec), IL-15 (Miltenyi Biotec) and TransAct (Miltenyi Biotec). After 24 h T cells were transduced with an MOI of 5 by adding the corresponding volume of lentiviral vector particles. On day 3 post activation the culture medium was removed and replaced by TexMACS medium (Miltenyi Biotec) supplemented with IL-7 (Miltenyi Biotec) and IL-15 (Miltenyi Biotec). Frequency of anti-tag CAR positive T cells as well as inducible T cells was indirectly analyzed on day 6 after transduction via flow cytometry determination of LNGFR expression using a LNGFR APC conjugate (Miltenyi Biotec). Transduced T cells were enriched for LNGFR positive cells on day 7 post transduction using MACSelect LNGFR MicroBeads (Miltenyi Biotec). Enrichment procedure was done according to suppliers protocol. Transduced T cells were used for functional assays on day 13 after activation.

### Example 2: Induction of anti-CD19 Fab-His₆ secretion and analysis of adapter concentrations

Lentiviral particles encoding for the drug-inducible cassette were manufactured as described in example 1.2. Drug-inducible T cells were generated as described in example 1.3. 1E4 LNGFR positive drug-inducible T cells were seeded in 100 µL TexMACS medium (Miltenyi Biotec) supplemented with IL-7 (Miltenyi Biotec) and IL-15 (Miltenyi Biotec) in a 96 round bottom well plate. Secretion of the His₆-tagged anti-CD19 Fab was induced by the addition of different concentrations of 4-OHT (0 - 500 nM, Sigma-Aldrich) in 100 µL µL TexMACS medium (Miltenyi Biotec) supplemented with IL-7 (Miltenyi Biotec) and IL-15 (Miltenyi Biotec) to each well. Untransduced T cells constitute the negative control. Cells were incubated at 37 °C and 5 % CO₂. His₆-tagged anti-CD19 Fab containing supernatant was harvested 48 h after the induction. CD19⁺ Raji cells were subsequently stained using T cell supernatant and anti-His-APC as secondary antibody. Therefore, 1E5 CD19⁺ Raji cells were seeded in a 96 round bottom well plate. Cells were pelleted at 300 g for 5 min and stained with 50 µL of the collected T cell supernatant for 10 min at 4°C. CD19⁺ Raji cells were washed twice with 200 µL CliniMACS buffer (Miltenyi Biotec) supplemented with 0.5% BSA (Miltenyi Biotec) (referred to as PEB) and incubated in 50 µL secondary staining mixture composed of the secondary antibody anti-His APC (Miltenyi Biotec) diluted in PEB for 10 min at 4°C. CD19⁺ Raji cells were washed with 200 µL PEB (300 g, 5 min). Cells were resuspended in 100 µL PEB for subsequent flow cytometric analysis. To exclude dead cells, Propidium iodide (PI; Miltenyi Biotec) was added to the stained cells directly before sample acquisition at the MACSQuant^{®} Analyzer 10 (Miltenyi Biotec). The determined mean fluorescent intensity in the APC channel was correlated to the Fab concentration by extrapolation from a standard curve. To generate the standard curve a defined concentration of a His₆-tagged anti-CD19-Fab (Miltenyi Biotec) was used.

Induction of His₆-tagged anti-CD19-Fab secretion by inducible T cells is strictly dependent on the presence of the inducer drug 4-OHT (Figure 3). With increasing concentrations of 4-OHT, increasing amounts of His₆-tagged anti-CD19-Fab is detected in the supernatant of the T cell culture as determined by the staining of CD19⁺ Raji cells and extrapolation from a standard curve.

### Example 3: Cytolytic activity of anti-His Adapter CAR T cells in co-culture with Raji cells in the presence of inducible anti-CD19 Fab T cells and the inducer drug 4-OHT.

Anti-His Adapter CAR T cell-mediated cytotoxicity against tumor cells was assessed by the quantification of living target cells using the MACSQuant^{®} Analyzer 10 (Miltenyi Biotec). Therefore, 1E4 GFP⁺ Raji cells were seeded in 50 µL TexMACS medium (Miltenyi Biotec) in a 96-well round-bottom plate. Anti-His Adapter CAR T cells were subsequently added at an E:T ratio of 2:1 in a volume of 50 µL TexMACS medium (Miltenyi Biotec). T cell numbers were adjusted to LNGFR expression implicating equal transduced and total T cell numbers in each well. The number of untransduced T cells was adjusted to total cell numbers. 1E4 anti-CD19 Fab inducible T cells that secrete the adapter molecule, a His-tagged anti-CD19 Fab, upon induction, were added to the respective wells. His-tagged anti-CD19 Fab secretion was induced by the addition of 100 nM 4-OHT (Sigma-Aldrich) in 50 µL TexMACS (Miltenyi Biotec) at the start of the assay. The plate was centrifuged at 300 g for 1 min and incubated at 37°C, 5% CO₂. The specific lysis of target cells was determined 6 days after co-culture set-up. Therefore, the plate was incubated at 4°C for 20 min to stop further target cell lysis. Next, living target cells were quantified via flow cytometry. Propidium iodide (PI; Miltenyi Biotec) was automatically added to the co-culture by the auto label function of the MACSQuant^{®} and 70 µL of each well were acquired using the acquisition mode high. Living Raji tumor cells were defined as PI⁻, GFP⁺ cells and specific lysis was calculated according to the following formula: % specific lysis = (1 - (Raji cell count [sample] / Raji cell count [target cell])) - 100%.

Specific lysis of CD19⁺ Raji cells was only detected in co-cultures of CD19⁺ Raji cells, adapter CAR T cells and inducible T cells in the presence of at least 1 nM 4-OHT (• symbol) (Figure 4). Maximal specific lysis was obtained following addition of at least 10 nM 4.OHT. A co-culture of untransduced T cells and CD19⁺ Raji in the presence of anti-CD19 Fab inducible T cells (■ symbol) as well as a co-culture of inducible anti-CD19 Fab T cells and CD19⁺ Raji (▲ symbol) served as negative control both indicating that lysis of tumor cells cannot be induced by the adapter molecule itself but requires the simultaneous presence of anti-His adapter CAR T cells.

### Example 4: Analysis of PD-1 expression by anti-His Adapter CAR T cells following co-culture with Raji cells in the presence of inducible anti-CD19 Fab T cells and the inducer drug 4-OHT.

To set up the co-culture, 1E4 GFP⁺ Raji cells were seeded in 50 µL TexMACS medium (Miltenyi Biotec) in a 96-well round-bottom plate. Anti-His Adapter CAR T cells were subsequently added at an E:T ratio of 2:1 in a volume of 50 µL TexMACS medium (Miltenyi Biotec). T cell numbers were adjusted to LNGFR expression implicating equal transduced and total T cell numbers in each well. The number of untransduced T cells was adjusted to total cell numbers. 1E4 anti-CD19 Fab inducible T cells that secrete the adapter molecule, a His-tagged anti-CD19 Fab, upon induction, were added to the respective wells. His-tagged anti-CD19 Fab secretion was induced by the addition of 100 nM 4-OHT (Sigma-Aldrich) in 50 µL TexMACS (Miltenyi Biotec) at the start of the assay. The plate was centrifuged at 300 g for 1 min and incubated at 37°C, 5% CO₂ for 2 days. Activation of T cells was analyzed by staining the cells of the co-culture with anti-PD-1-PEVio770, CD3-VioBlue, CD8-VioGreen and LNGFR-APC conjugates (Miltenyi Biotec). Therefore, the co-culture plate was centrifuged at 300 g for 5 min and cells were stained with 50 µL staining mixture composed of the PD-1 PE07, conjugate (Miltenyi Biotec) diluted in PEB for 10 min at 4°C. Cells were washed twice with 200 µL PEB (300 g, 5 min). Cells were resuspended in 100 µL PEB for subsequent flow cytometric analysis. To exclude dead cells, Propidium iodide (PI; Miltenyi Biotec) was added to the stained cells directly before sample acquisition at the MACSQuant^{®} Analyzer 10 (Miltenyi Biotec).

PD-1 expression on T cells was only detected in co-cultures of CD19⁺ Raji cells, adapter CAR T cells and inducible T cells in the presence of at least 10 nM 4-OHT and the frequency of PD-1 positive T cells increased with the concentration of 4-OHT (• symbol) (Figure 4). Maximal frequency of PD-1 positive cells was obtained following addition of at least 50 nM 4.OHT. A co-culture of untransduced T cells and CD19⁺ Raji in the presence of anti-CD19 Fab inducible T cells (■ symbol) as well as a co-culture of inducible anti-CD19 Fab T cells and CD19⁺ Raji (▲ symbol) served as negative control both indicating that activation (as shown by PD-1 expression) of T cells cannot be induced by the adapter molecule itself but requires the simultaneous presence of anti-His adapter CAR T cells.

## Claims

1. A system for inducible expression of an adapter in immune cells comprising
a) an inducible gene expression system comprising
I) a first nucleic acid comprising an inducible promoter operably linked to a second nucleic acid
II) said second nucleic acid encoding an adapter comprising
i) a first (poly)peptide, wherein said first (poly)peptide comprises an antigen binding domain that binds specifically to an antigen,
ii) a second (poly)peptide, wherein said second (poly)peptide binds to an antigen binding domain of a chimeric antigen receptor (CAR),
b) a third nucleic acid encoding said CAR specific for said second polypeptide of said adapter, wherein said CAR comprises
i) said antigen binding domain specific for said second (poly)peptide of said adapter
ii) a transmembrane domain
iii) an intracellular signaling domain.

2. The system according to claim 1, wherein said inducible gene expression system is an antigen-activated inducible gene expression system, and said antigen-activated inducible promoter is an antigen-activated promoter capable of driving expression of said adapter when a cell having said inducible gene expression system is activated by said antigen.

3. The system according to claim 1, wherein said inducible gene expression system is a drug-inducible expression system and said inducible promoter is a drug-inducible promoter, wherein said inducible gene expression system further comprises a nucleic acid encoding a synthetic transcription factor for said drug-inducible promoter, wherein when a drug is administered to a cell having said inducible gene expression system, the gene expression system is induced and the adapter is expressed.

4. The system according to claim 3, wherein said synthetic transcription factor comprises a DNA binding domain and drug-binding domain and an activation domain, wherein said synthetic transcription factor is activated by binding to said drug.

5. The system according to claim 3 or 4, wherein the level of expression of said adapter depends on the amount of drug administered to said cell, thereby allowing a tunable control of the expression of the adapter.

6. The system according to any one of claims 1 to 5, wherein said inducible gene expression system and said nucleic acid encoding said CAR specific for said second polypeptide of said adapter are present in one immune cell.

7. The system according to any one of claims 1 to 5, wherein said inducible gene expression system is present in a first immune cell and said nucleic acid encoding said CAR specific for said second polypeptide of said adapter is present in a second immune cell.

8. The system according to claim 7, wherein said first immune cell comprises a CAR specific for a further antigen, wherein said CAR comprises
i) said antigen binding domain specific for said further antigen
ii) a transmembrane domain
iii) an intracellular signaling domain,
and/or wherein said first immune cell comprises a TCR specific for a further antigen.

9. The system according to claim 7 or 8, wherein said first immune cell and said second immune cell are the same type of immune cell e.g. T cells or NK cells.

10. The system according to claim 7 or 8, wherein said first immune cell and said second immune cell are different type of immune cells, e.g. the first immune cell is a T cell and the second immune cell is a NK cell.

## Patentansprüche

1. System zur induzierbaren Expression eines Adapters in Immunzellen, umfassend
a) ein induzierbares Genexpressionssystem, umfassend
I) eine erste Nukleinsäure, die einen induzierbaren Promotor umfasst, der funktionell mit einer zweiten Nukleinsäure verbunden ist
II) wobei die zweite Nukleinsäure, die für einen Adapter kodiert, Folgendes umfasst:
i) ein erstes (Poly)peptid, wobei das erste (Poly)peptid eine Antigenbindungsdomäne umfasst, die spezifisch an ein Antigen bindet,
ii) ein zweites (Poly)peptid, wobei das zweite (Poly)peptid an eine Antigenbindungsdomäne eines chimären Antigenrezeptors (CAR) bindet,
b) eine dritte Nukleinsäure, die für den CAR kodiert, der für das zweite Polypeptid des Adapters spezifisch ist, wobei der CAR Folgendes umfasst:
i) die Antigenbindungsdomäne, die für das zweite (Poly)peptid des Adapters spezifisch ist
ii) eine Transmembrandomäne
iii)eine intrazelluläre Signaldomäne.

2. System nach Anspruch 1, wobei das induzierbare Genexpressionssystem ein Antigen-aktiviertes induzierbares Genexpressionssystem ist und der Antigenaktivierte induzierbare Promotor ein Antigen-aktivierter Promotor ist, der in der Lage ist, die Expression des Adapters zu steuern, wenn eine Zelle mit dem induzierbaren Genexpressionssystem durch das Antigen aktiviert wird.

3. System nach Anspruch 1, wobei das induzierbare Genexpressionssystem ein arzneimittelinduzierbares Expressionssystem ist und der induzierbare Promotor ein arzneimittelinduzierbarer Promotor ist, wobei das induzierbare Genexpressionssystem ferner eine Nukleinsäure umfasst, die für einen synthetischen Transkriptionsfaktor für den arzneimittelinduzierbaren Promotor kodiert, wobei, wenn ein Arzneimittel an eine Zelle verabreicht wird, die das induzierbare Genexpressionssystem aufweist, das Genexpressionssystem induziert wird und der Adapter exprimiert wird.

4. System nach Anspruch 3, wobei der synthetische Transkriptionsfaktor eine DNA-Bindungsdomäne, eine Arzneimittelbindungsdomäne und eine Aktivierungsdomäne umfasst, wobei der synthetische Transkriptionsfaktor durch Bindung an das Arzneimittel aktiviert wird.

5. System nach Anspruch 3 oder 4, wobei das Ausmaß der Expression des Adapters von der Menge des der Zelle verabreichten Arzneimittels abhängt, wodurch eine einstellbare Steuerung der Expression des Adapters ermöglicht wird.

6. System nach einem der Ansprüche 1 bis 5, wobei das induzierbare Genexpressionssystem und die Nukleinsäure, die für den CAR kodiert, der für das zweite Polypeptid des Adapters spezifisch ist, in einer Immunzelle vorhanden sind.

7. System nach einem der Ansprüche 1 bis 5, wobei das induzierbare Genexpressionssystem in einer ersten Immunzelle vorhanden ist und die Nukleinsäure, die für den CAR kodiert, der für das zweite Polypeptid des Adapters spezifisch ist, in einer zweiten Immunzelle vorhanden ist.

8. System nach Anspruch 7, wobei die erste Immunzelle einen CAR umfasst, der für ein weiteres Antigen spezifisch ist, wobei der CAR Folgendes umfasst:
i) die Antigenbindungsdomäne, die für das weitere Antigen spezifisch ist,
ii) eine Transmembrandomäne,
iii) eine intrazelluläre Signaldomäne,
und/oder wobei die erste Immunzelle einen TCR umfasst, der für ein weiteres Antigen spezifisch ist.

9. System nach Anspruch 7 oder 8, wobei die erste Immunzelle und die zweite Immunzelle der gleiche Art von Immunzelle sind, z. B. T-Zellen oder NK-Zellen.

10. System nach Anspruch 7 oder 8, wobei die erste Immunzelle und die zweite Immunzelle unterschiedliche Arten von Immunzellen sind, z. B. die erste Immunzelle ist eine T-Zelle und die zweite Immunzelle ist eine NK-Zelle.

## Revendications

1. Système pour l'expression inductible d'un adaptateur dans des cellules immunitaires comprenant
a) un système d'expression de gène inductible comprenant
I) un premier acide nucléique comprenant un promoteur inductible lié opérationnellement à un second acide nucléique
II) ledit second acide nucléique codant pour un adaptateur comprenant
i) un premier (poly)peptide, ledit premier (poly)peptide comprenant un domaine de liaison à un antigène qui se lie spécifiquement à un antigène,
ii) un second (poly)peptide, ledit second (poly)peptide se liant à un domaine de liaison à un antigène d'un récepteur d'antigène chimérique (CAR),
b) un troisième acide nucléique codant pour ledit CAR spécifique pour ledit second polypeptide dudit adaptateur, ledit CAR comprenant
i) ledit domaine de liaison à un antigène spécifique pour ledit second (poly)peptide dudit adaptateur
ii) un domaine transmembranaire
iii) un domaine de signalisation intracellulaire.

2. Système selon la revendication 1, dans lequel ledit système d'expression de gène inductible est un système d'expression de gène inductible activé par antigène et ledit promoteur inductible activé par antigène est un promoteur activé par antigène capable de diriger l'expression dudit adaptateur quand une cellule ayant ledit système d'expression de gène inductible est activée par ledit antigène.

3. Système selon la revendication 1, dans lequel ledit système d'expression de gène inductible est un système d'expression inductible par médicament et ledit promoteur inductible est un promoteur inductible par médicament, ledit système d'expression de gène inductible comprenant en outre un acide nucléique codant pour un facteur de transcription synthétique pour ledit promoteur inductible par médicament, quand un médicament est administré à une cellule ayant ledit système d'expression de gène inductible, le système d'expression de gène étant induit et l'adaptateur étant exprimé.

4. Système selon la revendication 3, dans lequel ledit facteur de transcription synthétique comprend un domaine de liaison à l'ADN et un domaine de liaison à un médicament et un domaine d'activation, ledit facteur de transcription synthétique étant activé par liaison audit médicament.

5. Système selon la revendication 3 ou 4, dans lequel le niveau d'expression dudit adaptateur dépend de la quantité de médicament administrée à ladite cellule, permettant ainsi un contrôle accordable de l'expression de l'adaptateur.

6. Système selon l'une quelconque des revendications 1 à 5, dans lequel ledit système d'expression de gène inductible et ledit acide nucléique codant pour ledit CAR spécifique pour ledit second polypeptide dudit adaptateur sont présents dans une cellule immunitaire.

7. Système selon l'une quelconque des revendications 1 à 5, dans lequel ledit système d'expression de gène inductible est présent dans une première cellule immunitaire et ledit acide nucléique codant pour ledit CAR spécifique pour le second polypeptide dudit adaptateur est présent dans une seconde cellule immunitaire.

8. Système selon la revendication 7, dans lequel ladite première cellule immunitaire comprend un CAR spécifique pour un autre antigène, ledit CAR comprenant
i) ledit domaine de liaison à un antigène spécifique pour l'autre antigène
ii) un domaine transmembranaire
iii) un domaine de signalisation intracellulaire
et/ou ladite première cellule immunitaire comprenant un TCR spécifique pour un autre antigène.

9. Système selon la revendication 7 ou 8, dans lequel ladite première cellule immunitaire et ladite seconde cellule immunitaire sont le même type de cellule immunitaire par exemple des lymphocytes T ou des cellules NK.

10. Système selon la revendication 7 ou 8, dans lequel ladite première cellule immunitaire et ladite seconde cellule immunitaire sont des types différents de cellules immunitaires, par exemple la première cellule immunitaire est un lymphocyte T et la seconde cellule immunitaire est une cellule NK.
